# EUROPEAN PATENT SPECIFICATION

(11) **EP 1 450 755 B1**
(45) Date of publication and mention of the grant of the patent: **13.08.2008**
(21) Application number: 02779533.5
(22) Date of filing: 05.11.2002
(51) Int. Cl.: A61K 8/81, A61K 8/91, A61Q 5/06

(54) **HAIR STYLING COMPOSITION**
HAARFORMUNGSZUSAMMENSETZUNGEN
COMPOSITION DE COIFFURE

(30) Priority: 03.12.2001 GB 0128951
(43) Date of publication of application: 01.09.2004
(73) Proprietor: Unilever PLC, London Greater London EC4P 4BQ (GB); Unilever N.V., 3013 AL Rotterdam (NL)
(72) Inventor: ANDREWS, Darren P, Unilever Res. Port Sunlight, Wirral, Merseyside CH63 3JW (GB); KUZNITZ, Matthew F, Unilever Home & Pers. Care USA, Chicago, IL 60008 (US); PRATLEY, Stuart K, Unilever Research Port Sunlight, Wirral, Merseyside CH63 3JW (GB); SENGUPTA, Tapashi, Unilever Home & Pers. Care USA, Chicago, IL 60008 (US)
(86) International application number: PCT/EP2002/012378
(87) International publication number: WO 2003/047538

(56) References cited:
- WO-A-00/21493
- WO-A-00/49998
- DE-B- 1 077 430

## Description

### Field of the Invention

The present invention relates to hair styling compositions, for example creams, gels and especially aerosol hair styling mousse compositions, and to methods of styling hair using the compositions.

### Background and Prior Art

Style creation products such as hair styling mousses provide human hair with a temporary set which can be removed by water or by shampooing, and function by applying a thin film of a resin or gum onto the hair to adhere adjacent hairs together so that they retain the particular shape or configuration at the time of application.

EP 818 190 describes how an emulsion polymerised silicone material having a particular, defined level of cross-linking, and which is cross-linked in emulsion form can be incorporated into a hair styling composition, such as a mousse, gel or cream, to give a formulation which delivers excellent style creation and longevity, whilst leaving the hair soft and natural. An important feature of these systems is the phase behaviour of the silicone, which is said to form a separate high viscosity aggregated phase in the composition. This phase behaviour is considered to be key to effective style creation.

A problem with "aggregating" systems as described in EP 818 190 is that they can tend to gel and form lumps under conditions of prolonged, high temperature storage.

International patent application no PCT/EP99/07427 discloses the use of certain non-ionic surfactants of specified HLB value for use in compositions comprising silicone materials of the type described in EP 818 190. The resulting systems do not form a separate high viscosity aggregated phase and yet nevertheless deliver effective style creation.

Graft copolymers of polyvinyl alcohol on polyalkylene glycols are known. For example, DE 1 077 430 describes a process for the preparation of graft polymers of vinyl esters on polyalkylene glycols. The preparation of graft copolymers of polyvinyl alcohol on polyalkylene glycols by hydrolysis of the vinyl esters is described in DE 1 094 457 and DE 1 081 229. The use of water-soluble or water-dispersible polymerisates, obtained by the polymerisation of a vinyl ester in the presence of a polyether, as coating agents, binding agents or film-forming auxiliary agents in pharmaceutical formulations, is described in WO 00/18375. The use of the polymers in formulations of hair cosmetics is described in WO 00/49998, published on 31 August 2000. Cross-linking of the polymers is described in that document. However, no examples are given of formulations containing the cross-linked polymers or of components that such compositions might contain.

Our copending application WO 98/14504 discloses the surprising finding that hair styling compositions containing a copolymer of polyvinyl alcohol and polyalkylene glycols, such as are obtainable by graft polymerisation, in addition to a cross-linked silicone, have improved feel and/or hold compared to compositions which do not contain the copolymer. The compositions comprise at least 0.1% by weight of a non-rigid emulsion polymerised cross-linked silicone polymer.

At least as important as the properties of the finished style are those properties of the formulation that allow the style to be created. Obtaining the correct balance of styling properties to styled properties is a difficult problem and often the result is a trade-off of one against the other.

The present invention is based on the unexpected finding that improved hair styling compositions may be obtained by using cross-linked polymers having specific properties, together with specific cationic polymers. We have found that, by using cross-linked graft copolymers with particular molecular weight properties, extremely good styling properties are obtained whilst maintaining a high quality finished style. In particular, it has been found that particularly effective styling compositions rely on the cross-linked polymer having a defined polydispersity and the presence of a cationic polymer.

### SUMMARY OF THE INVENTION

The present invention provides a hair styling composition comprising:
(i) from 0.01% to 10% by weight of a copolymer having a backbone comprising a polyether and, depending from said backbone, a plurality of poly(vinyl ester) groups, wherein at least some of the ester groups are hydrolysed to the corresponding alcohol, and wherein the copolymer is crosslinked and has a polydispersity of at least 5;
(ii) from 0.01% to 10% by weight of a cationic hair styling polymer;
(iii) optionally, a cross linked silicone polymer in an amount of less than 0.1% by weight; and
(iv) water.

In another aspect, the invention provides a method of styling hair which comprises applying to the hair a composition of the invention.

A yet further aspect of the invention is the use of the compositions of the invention for the styling of hair.

### DETAILED DESCRIPTION

Compositions of the invention surprisingly deliver excellent style as well as sensory feel to the hair compared to the compositions of the prior art. Unexpectedly, the compositions of the invention can be effective, even though they can contain little or no cross-linked silicone polymer.

Compositions of the invention preferably comprise a total amount of copolymer (i) and cationic hair styling polymer (ii) of less than 10% by weight (more preferably less than 10% by weight) of the total weight of the composition, more preferably 1% to 4.5% by weight, even more preferably 2% to 4% by weight.

In the compositions of the invention, the weight ratio of copolymer (i) to cationic hair styling polymer (ii) is preferably 10:1 to 1:10, more preferably 5:1 to 1:5, even more preferably from 2:1 to 1:2, most preferably about 1:1.

Compositions of the invention may have one or more advantages including improved hold and/or body and/or volume of the styled hair and/or increased ease of style creation and/or longer lasting style and/or natural feel. In addition, films formed by the compositions of the invention on hair may have improved flexibility and/or toughness.

### Copolymer Having A Polyether Backbone

The compositions of the invention comprise from 0.01% to 10% (preferably 0.1% to 5%, more preferably 1% to 2% such as 1.5%) by weight of a copolymer having a backbone (or main chain; the terms backbone and main chain are used synonymously herein) comprising a polyethylenglycol and, depending from the backbone, a plurality of poly (vinyl ester) groups. At least 50% of the ester groups are hydrolysed to the corresponding alcohol, preferably at least 90%, most preferably at least 95% of the groups are hydrolysed to the corresponding alcohol. The poly (vinyl ester) chains optionally contain other functional groups in and/or on the polymer chain, such as, for example, amide and/or keto groups. The copolymer comprises a polyethyleneglycol backbone.

The copolymer is preferably polyethyleneglycol-copolyvinylalcohol having polyvinylalcohol groups bound to the polyethyleneglycol i.e., substantially all of the poly (vinyl ester) groups are preferably hydrolysed in the copolymers used in the compositions of the invention.

The copolymer can be produced by methods which are well-known to those skilled in the art. For example, the copolymers are obtainable by graft polymerisation. In a method comprising graft polymerisation, poly (vinyl ester) groups are preferably grafted onto a polyether and are subsequently hydrolysed to convert at least some of the ester groups to the corresponding alcohol. For example, DE 1 077 430 describes a process for the preparation of graft polymers of vinyl esters on polyalkylene glycols. The preparation of graft copolymers of polyvinyl alcohol on polyalkylene glycols by hydrolysis of the vinyl esters is described in DE 1 094 457 and DE 1 081 229.

The weight average molecular weight of the polyether is preferably from 1 to 100 kDa.

Preferred copolymers for use in compositions of the invention have a molar ratio of polyether to total hydrolysed and unhydrolysed poly (vinyl ester) groups, ie, poly(vinyl ester) and polyvinylalcohol groups, in the range of from about 95:5 to 5:95, more preferably about 30:70 to about 70:30. Typically, such copolymers have a weight ratio of polyether to total poly(vinyl ester) and polyvinylalcohol groups of about 40:60.

The copolymer (i) is cross-linked. Suitable cross-linking agents are those compounds which can bind to two or more polyether, poly (vinyl ester) and/or poly (vinyl alcohol) chains and include, for example, allyl ethers of polyhydroxyl compounds such as pentaerythritol triallyl ether. The copolymer preferably comprises 0.1% to 1% by weight cross-linker (eg, 0.2% to 0.6% by weight).

The copolymer has a polydispersity of at least 5, preferably from 5 to 40, more preferably 10 to 40, even more preferably 15 to 40, most preferably 15 to 38. Polydispersity (Mw/Mn) is defined as the ratio of weight average molecular weight (Mw) to number average molecular weight (Mn) and can be determined by gel permeation chromatography (GPC) according to the methods described herein in the Examples section.

The copolymer which is used in the invention, and the methods for its preparation, are further described below.

Broadly, the copolymer which may be used in the invention is obtainable by free radical polymerisation of:
a) at least one vinyl ester; in the presence of
b) polyether-containing compounds; and
c) at least one other copolymerisable monomer;
followed by subsequent at least partial hydrolysis of the ester functions of the original monomers (a). Copolymerisable monomer c) may constitute the cross-linking agent.

In the preparation of the polymers used according to the invention, grafting onto the polyether-containing compounds (b) can result during the polymerization, which can lead to advantageous properties for the polymers. However, mechanisms other than grafting are also possible.

Depending on the degree of grafting, the polymers used according to the invention are taken to mean pure graft polymers and also mixtures of the abovementioned graft polymers with non-grafted polyether-containing compounds and homo- or copolymers of the monomers (a) and (c).

Polyether-containing compounds (b) which can be used are either polyalkylene oxides, based on ethylene oxide, propylene oxide, butylene oxide and other alkylene oxides, or polyglycerol. Depending on the type of monomer units, the polymers contain the following structural units.
-(CH₂)₂-O-, -(CH₂)₃-O-, -(CH₂)₄-O-, -CH₂-CH (R⁶) -O-, -CH₂-CHOR⁷- -CH₂-O-
where
R⁶ is C₁ - C₂₄- alkyl;
R⁷ is hydrogen, C₁- C₂₄ - alkyl, R⁶-C (=O) -, R⁶-NH-C (=O) - .

The structural units can either be homopolymers or random copolymers and block copolymers.

For polyether (b), preference is given to using polymers of the formula I, 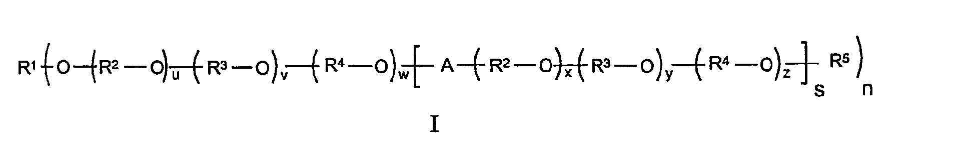 in which the variables, independently of one another, have the following meanings:
R¹ is hydrogen, C₁ - C₂₄ - alkyl, R⁶-C (=O) -, R⁶-NH-C(=O)-, polyalcohol radical;
R⁵ is hydrogen, C₁ - C₂₄- alkyl, R⁶-C (=O) -, R⁶-NH-C (=O) - ;
R² to R⁴ are -(CH₂)₂ - , -(CH₂)₃-, -(CH₂)₄-, -CH₂-CH (R⁶) - , -CH₂-CHOR⁷-CH₂- ;
R⁶ is C₁- C₂₄- alkyl;
R⁷ is hydrogen, C₁ - C₂₄-alkyl, R⁶-C (=O) -, R⁶-NH-C(=O)-;
A is -C (=O) -O, -C(=O)-B-C(=O)-O, -C(=O)-NH-B-NH-C(=O)-O;
B is - (CH2)t-, arylene (eg, phenylene) , optionally substituted;
n is from 1 to 1000;
s is from 0 to 1000;
t is from 1 to 12;
u is from 1 to 5000;
v is from 0 to 5000;
w is from 0 to 5000;
x is from 0 to 5000;
y is from 0 to 5000;
z is from 0 to 5000.

The terminal primary hydroxyl groups of the polyethers prepared on the basis of polyalkylene oxides, and the secondary hydroxyl groups of polyglycerol can in this connection either be present in free unprotected form, or be etherified with alcohols of chain length C₁-C₂₄, or esterified with carboxylic acids of chain length C₁-C₂₄, or reacted with isocyanates to give urethanes.

Alkyl radicals which may be mentioned for R¹ and R⁵ to R⁷ are branched or unbranched C₁-C₂₄- alkyl chains, preferably methyl, ethyl, n-propyl, 1-methylethyl, n-butyl, 1-methylpropyl, 2-methylpropyl, 1, 1-dimethylethyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2 -methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-heptyl, 2-ethylhexyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl or n-eicosyl.

Preferred representatives of the abovementioned alkyl radicals which may be mentioned are branched or unbranched C₁-C₁₂- alkyl chains, with C₁-C₆-alkyl chains being particularly preferred.

The molecular weight of the polyethers is less than 1000000 (number average), preferably in the range from 300 to 100000, particularly preferably in the range from 500 to 50000, very particularly preferably in the range from 800 to 40000.

Homopolymers of ethylene oxide or copolymers carrying an ethylene oxide content of from 40 to 99% by weight are advantageously used. For the ethylene oxide polymers to be used in preference, the content of copolymerized ethylene oxide is thus from 40 to 100 mol%. Suitable comonomers for these copolymers are propylene oxide, butylene oxide, and/or iso-butylene oxide. Suitable examples are copolymers of ethylene oxide and propylene oxide, copolymers of ethylene oxide and butylene oxide, and copolymers of ethylene oxide, propylene oxide and at least one butylene oxide. The ethylene oxide content of the copolymers is preferably from 40 to 99 mol%, the propylene oxide content is from 1 to 60 mol% and the content of butylene oxide in the copolymers is from 1 to 30 mol%. As well as straight-chain homo- or copolymers, it is also possible to use branched homo- or copolymers as polyether-containing compounds (b).

Branched polymers can be prepared by adding ethylene oxide and optionally also propylene oxide and/or butylene oxides to, for example, polyalcohol radicals, e.g. to pentaerythritol, glycerol, or to sugar alcohols such as D-sorbitol and D-mannitol, but also to polysaccharides such as cellulose and starch. The alkylene oxide units can be randomly distributed or be in the form of blocks within the polymer.

It is, however, also possible to use polyesters of polyalkylene oxides and aliphatic or aromatic dicarboxylic acids, e.g. oxalic acid, succinic acid, adipic acid and terephthalic acid having molar masses of from 1500 to 25000, as described, for example, in EP-A-0 743 962, as well as polyether-containing compounds. In addition, it is also possible to use polycarbonates by reaction of polyalkylene oxides with phosgene or carbonates such as, for example, diphenyl carbonate, and polyurethanes by reaction of polyalkylene oxides with aliphatic and aromatic diisocyanates.

Particularly preferred polyethers (b) are polymers of the formula I having an average molecular weight of from 300 to 100000 (number average), in which the variables independently of one another have the following meanings:
R¹ is hydrogen, C₁ - C₁₂-alkyl, R⁶-C (=O) -, R⁶-NH-C (=O) -, polyalcohol radical;
R⁵ is hydrogen, C₁-C₁₂ - alkyl, R⁶-C (=O) -, R⁶-NH-C(=O)- ;
R² to R⁴ are - (CH₂) ₂ -, -(CH₂)₃-, -(CH₂)₄-, -CH₂-CH (R⁶) - , -CH₂-CHOR⁷- CH₂-;
R⁶ is C₁-C₁₂ - alkyl;
R⁷ is hydrogen, C₁-C₁₂- alkyl, R⁶-C(=O)-, R⁶-NH-C (=O) -;
n is from 1 to 8;
s is 0;
u is from 2 to 2000;
v is from 0 to 2000;
w is from 0 to 2000.

Very particularly preferred polyethers (b) are polymers of the formula I having an average molecular weight of from 500 to 50000 (number average), in which the variables independently of one another have the following meanings:
R¹ is hydrogen, C₁-C₆ - alkyl, R⁶-C(=O)-, R⁶-NH-C (=O) -;
R⁵ is hydrogen, C₁-C₆- alkyl, R⁶-C(=O)-, R⁶-NH-C (=O) -;
R² to R⁴ are -(CH₂)₂-, -(CH₂)₃-, -(CH₂)₄- , -CH₂-CH(R⁶)-, -CH₂-CHOR⁷-CH₂-;
R⁶ is C₁-C₆ - alkyl;
R⁷ is hydrogen, C₁ - C₆ - alkyl, R⁶-C (=O) -, R⁶-NH-C (=O) - ;
n is 1;
s is 0;
u is from 5 to 500;
v is from 0 to 500;
w is from 0 to 500.

The polyethers used may also be silicone derivatives. Suitable silicone derivatives are the compounds known under the INCI name 'dimethicone copolyols' or silicone surfactants, such as, for example, those available under the tradenames Abil^{®} (T. Goldschmidt) , Alkasil^{®} (Rhone-Poulenc), Silicone Polyol Copolymer^{®} (Genesee), Belsil^{®} (Wacker), Silwet^{®} (Witco, Greenwich, CT, USA) or Dow Corning (Dow Corning). These include compounds having the CAS numbers 64365-23-7; 68937-54-2; 68938-54-5; 68937-55-3.

Silicones are generally used in hair cosmetics to improve the feel. The use of polyether-containing silicone derivatives as polyether (b) in the polymers according to the invention can, therefore, additionally lead to an improvement in the feel of the hair.

Preferred representatives of those polyether-containing silicone derivatives are those which contain the following structural elements: 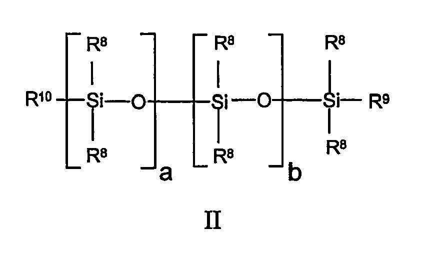 where:
R⁹ = CH₃ or 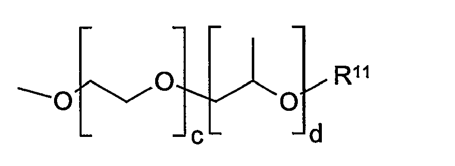 R¹⁰ = CH₃ or R⁹
R¹¹ = H or CH₃ or 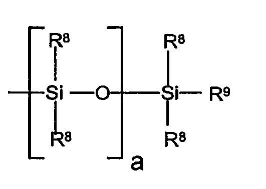 or

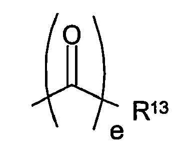

R¹³ is a C₁-C₄₀ organic radical which can contain amino, carboxyl or sulfonate groups, or where e = 0, is also the anion of an inorganic acid,
and where the radicals R⁸ can be identical or different, and either come from the group of aliphatic hydrocarbons having from 1 to 20 carbon atoms, or are cyclic aliphatic hydrocarbons having from 3 to 20 carbon atoms, or are of an aromatic nature, or are identical to R¹², where: 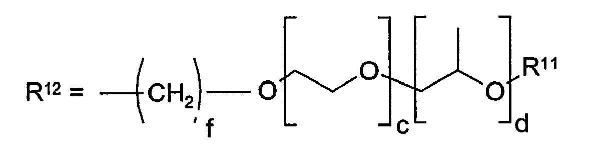 with the proviso that at least one of the radicals R⁸, R⁹ or R¹⁰, is a polyalkylene oxide-containing radical as defined above, and f is an integer from 1 to 6,
a and b are integers such that the molecular weight of the polysiloxane block is between 300 and 30000,
c and d can be integers between 0 and 50, with the proviso that the sum c + d is greater than 0, and e is 0 or 1.

Radicals R⁹ and R¹² are preferably those in which the sum of c+d is between 5 and 30.

The R⁸ groups are preferably chosen from the following list: methyl, ethyl, propyl, butyl, isobutyl, pentyl, isopentyl, hexyl, octyl, decyl, dodecyl and octadecyl, cycloaliphatic radicals, specifically cyclohexyl, aromatic groups, specifically phenyl or naphthyl, mixed aromatic-aliphatic radicals such as benzyl or phenyl ethyl and tolyl and xylyl and R¹².

Particularly suitable R¹¹ radicals are those in which R¹¹ = (_{CO})e₋R₁³ where R¹³ is any desired alkyl, cycloalkyl or aryl radical which has between 1 and 40 carbon atoms and which can carry other ionogenic groups such as NH₂, COOH, S0₃H.

Preferred inorganic R¹³ radicals are those where e = 0, and which contain phosphate and sulfate.

Particularly preferred polyether-containing silicone derivatives are those with the structure:

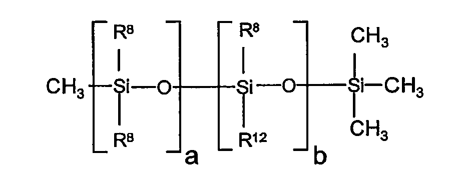

In addition, copolymers of alkylene oxides with ethylenically unsaturated monomers, such as, for example, polyalkylene oxide (meth)acrylates, polyalkylene oxide vinyl ethers, polyalkylene oxide (meth)acrylamides, polyalkylene oxide allylamides or polyalkylene oxide vinylamides can also be used as polyether (b). It is, of course, also possible to use copolymers of alkylene oxides with other ethylenically unsaturated monomers.

It is also possible to use polyether-containing compounds (b) derived from polyethyleneimines and alkylene oxides. In this case, the alkylene oxides used are preferably ethylene oxide, propylene oxide, butylene oxide and mixtures thereof, particularly preferably ethylene oxide. Polyethyleneimines which can be used are polymers having number-average molecular weights of from 300 to 20000, preferably from 500 to 10000, very particularly preferably from 500 to 5000. The weight ratio between alkylene oxide and polyethyleneimine is in the range from 100 _{:} 1 to 0.1 _{:} 1, preferably in the range from 50 _{:} 1 to 0.5 _{:} 1, very particularly preferably in the range from 20 _{:} 1 to 0.5 _{:} 1.

For polymerization in the presence of the polyether (b), monomer (a) may be drawn from any of the following free radical polymerisable monomers:
vinyl esters of aliphatic, saturated or unsaturated C₁-C₂₄ carboxylic acids, for example: formic acid, acetic acid, propionic acid, butyric acid, valeric acid, isovaleric acid, caproic acid, caprylic acid, capric acid, undecylenic acid, lauric acid, myristic acid, palmitic acid, palmitoleic acid, stearic acid, oleic acid, arachidic acid, behenic acid, lignoceric acid, cerotinic acid and melissic acid.

Vinyl esters of the abovementioned C₁-C₁₂ carboxylic acids are preferred with C₁ - C₆ carboxylic acids being particularly preferred. Vinyl acetate is very particularly preferred.

It is, of course, also possible to copolymerize mixtures of the respective (a) monomers.

Monomer (a) can, in addition, also be used in admixture with one or more ethylenically unsaturated copolymerizable comonomers (c), where the content of these additional monomers should be limited to a maximum of 50% by weight. Preference is given to contents of from 0 to 20% by weight. The term ethylenically unsaturated means that the monomers have at least one free-radically polymerizable carbon-carbon double bond which can be mono-, di-, tri- or tetrasubstituted.

The preferred ethylenically unsaturated comonomer (c) can be described by the following formula:

X -C (O) CR¹⁵=CHR¹⁴

where
X is chosen from the group of radicals -OH, -OM, -OR¹⁶, NH₂, NHR¹⁶, N(R¹⁶)₂;
M is a cation chosen from the group consisting of: Na⁺, K⁺, Mg⁺⁺, Ca⁺⁺, Zn⁺⁺, NH₄⁺, alkyl ammonium, dialkylammonium, trialkylammonium and tetraalkylammonium.

The R¹⁶ radicals can be identical or different and are chosen from the group consisting of: -H, C₁-C₄₀ linear or branched alkyl radicals, N, N-dimethylaminoethyl, 2-hydroxyethyl, 2-methoxyethyl, 2-ethoxyethyl, hydroxypropyl, methoxypropyl or ethoxypropyl.

R¹⁵ and R¹⁴ are chosen, independently, from the group consisting of: -H, C₁-C₈ linear or branched alkyl chains, methoxy, ethoxy, 2-hydroxyethoxy, 2-methoxyethoxy and 2-ethoxyethyl.

Representative, but non-limiting, examples of suitable (c) monomers are: acrylic or methacrylic acid and salts, and esters and amides thereof. The salts can be derived from any desired non-toxic metal, ammonium or substituted ammonium counter-ions.

The esters can be derived from: C₁-C₄₀ linear, C₃-C₄₀ branched or C₃-C₄₀ carbocyclic alcohols, from polyfunctional alcohols, having from 2 to about 8 hydroxyl groups, such as ethylene glycol, hexylene glycol, glycerol and 1, 2, 6-hexanetriol, from aminoalcohols or alcohol ethers such as methoxyethanol and ethoxyethanol, (alkyl)polyethylene glycols, (alkyl)polypropylene glycols or ethoxylated fatty alcohols, for example C₁₂-C₂₄ fatty alcohols reacted with 1 to 200 ethylene oxide units.

Also suitable are N,N-dialkylaminoalkyl acrylates and methacrylates and N-dialkylaminoalkylacryl- and - methacrylamides of general formula (III). 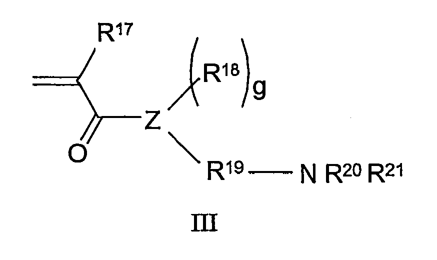 where
- R¹⁷ =: H, alkyl having from 1 to 8 carbon atoms,
- R¹⁸ =: H, methyl,
- R¹⁹=: alkylene having from 1 to 24 carbon atoms, optionally substituted by alkyl,
- R²⁰, R²¹ =: C₁ - C₄₀ alkyl radical,
- Z =: nitrogen when g = 1, or oxygen when g = 0.

The amides can be unsubstituted, N-alkyl- or N-alkylamino-mono-substituted or N,N-dialkyl-substituted or N,N-dialkylamino- di-substituted, where the alkyl or alkylamino groups are derived from C₁-C₄₀ linear, C₃-C₄₀ branched, or C₃ -C₄₀ carbocyclic units. In addition, the alkylamino groups can be quaternized.

Preferred examples of comonomers described by formula III are N,N-dimethylaminomethyl (meth)acrylate, N,N-diethylaminomethyl (meth)acrylate, N,N-dimethylaminoethyl (meth)acrylate, N,N-diethylaminoethyl (meth)acrylate, N-[3-(dimethylamino)propyl]methacrylamide and N-[3-(dimethylamino)propyl]acrylamide.

Monomers which can likewise be used as comonomer (c) are: substituted acrylic acids and salts, esters and amides thereof, where the substituents on the carbon atoms are in the two or three position of the acrylic acid, and are chosen, independently, from the group consisting of C₁-C₄ alkyl, -CN, and COOH. Methacrylic acid, ethacrylic acid and 3-cyanoacrylic acid are particularly preferred. Salts, esters and amides of these substituted acrylic acids can be chosen as described above for the salts, esters and amides of acrylic acid.

Other suitable group (c) monomers are: allyl esters of C₁-C₄₀ linear, C₃-C₄₀ branched or C₃ - C₄₀ carbocyclic carboxylic acids, vinyl or allyl halides, preferably vinyl chloride and allyl chloride, vinyl ethers, preferably methyl, ethyl, butyl or dodecyl vinyl ether, vinylformamide, vinylmethylacetamide, vinylamine; vinyllactams, preferably vinylpyrrolidone and vinylcaprolactam, vinyl- or allyl- substituted heterocyclic compounds, preferably vinylpyridine, vinyloxazoline and allylpyridine.

Also suitable are N-vinylimidazoles of general formula IV, in which R²² to R²⁴ are chosen, independently, from hydrogen, C₁-C₄ - alkyl or phenyl.

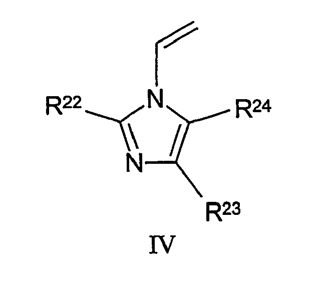

Other suitable (c) comonomers are diallylamines with general formula (V) 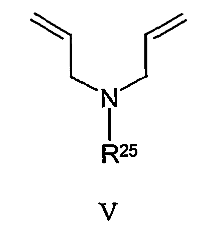 where R²⁵ - C₁-C₂₄ alkyl.

Other suitable (c) comonomers are vinylidene chloride and hydrocarbons having at least one carbon-carbon double bond, preferably chosen from: styrene, alpha-methylstyrene, tert- butylstyrene, butadiene, isoprene, cyclohexadiene, ethylene, propylene, 1-butene, 2-butene, isobutylene, vinyltoluene, and mixtures of these monomers.

Particularly suitable (c) comonomers are: acrylic acid, methacrylic acid, ethyl acrylic acid, methyl acrylate, ethyl acrylate, propyl acrylate, n-butyl acrylate, iso-butyl acrylate, t-butyl acrylate, 2-ethylhexyl acrylate, decyl acrylate, methyl methacrylate, ethyl methacrylate, propyl methacrylate, n-butyl methacrylate, iso-butyl methacrylate, t-butyl methacrylate, 2-ethylhexyl methacrylate, decyl methacrylate, methyl ethacrylate, ethyl ethacrylate, n-butyl ethacrylate, iso-butyl ethacrylate, t-butyl ethacrylate, 2-ethylhexyl ethacrylate, decyl ethacrylate, stearyl (meth)acrylate, 2,3-dihydroxypropyl acrylate, 2,3-dihydroxypropyl methacrylate, 2-hydroxyethyl acrylate, hydroxypropyl acrylates, 2-hydroxyethyl methacrylate, 2-hydroxyethyl ethacrylate, 2-methoxyethyl acrylate, 2-methoxyethyl methacrylate, 2-methoxyethyl ethacrylate, 2-ethoxyethyl methacrylate, 2-ethoxyethyl ethacrylate, hydroxypropyl methacrylates, glyceryl monoacrylate, glyceryl monomethacrylate, polyalkylene glycol (meth)acrylates, unsaturated sulfonic acids such as, for example, acrylamidopropane sulfonic acid;
acrylamide, methacrylamide, ethacrylamide, N-methylacrylamide, N,N-dimethylacrylamide, N-ethylacrylamide, N-isopropylacrylamide, N-butylacrylamide, N-t-butylacrylamide, N-octylacrylamide, N-t-octylacrylamide, N-octadecylacrylamide, N-phenylacrylamide, N-methylmethacrylamide, N-ethylmethacrylamide, N-dodecylmethacrylamide, 1-vinylimidazole, 1-vinyl-2-methylvinylimidazole, N,N-dimethylaminomethyl (meth)acrylate, N,N-diethylaminomethyl (meth)acrylate, N,N-dimethylaminoethyl (meth)acrylate, N,N-diethylaminoethyl (meth)acrylate, N,N-dimethylaminobutyl (meth)acrylate, N,N-diethylaminobutyl (meth)acrylate, N,N-dimethylaminohexyl (meth)acrylate, N,N-dimethylaminooctyl (meth)acrylate, N,N-dimethylaminododecyl (meth)acrylate, N-[3-(dimethylamino)propyl]methacrylamide, N-[3-(dimethylamino)propyl]acrylamide, N-[3(-dimethylamino)butyl]methacrylamide, N-[8-(dimethylamino)octyl]methacrylamide, N-[12-(dimethylamino)dodecyl]methacrylamide, N-[3-(diethylamino)propyl]methacryamide, N-[3-(diethylamino)propyl]acrylamide;
maleic acid, fumaric acid, maleic anhydride and its half-esters, crotonic acid, itaconic acid, diallyldimethylammonium chloride, vinyl ethers (for example: methyl, ethyl, butyl or dodecyl vinyl ether), vinyl formamide, vinylmethylacetamide, vinylamine; methyl vinyl ketone, maleimide, vinylpyridine, vinylimidazole, vinylfuran, styrene, styrene sulfonate, allyl alcohol, and mixtures thereof.

Of these, particular preference is given to: acrylic acid, methacrylic acid, maleic acid, fumaric acid, crotonic acid, maleic anhydride and its half-esters, methyl acrylate, methyl methacrylate, ethyl acrylate, ethyl methacrylate, n-butyl acrylate, n-butyl methacrylate, t-butyl acrylate, t-butyl methacrylate, iso-butyl acrylate, iso-butyl methacrylate, 2-ethylhexyl acrylate, stearyl acrylate, stearyl methacrylate, N-t-butylacrylamide, N-octylacrylamide, 2-hydroxyethyl acrylate, hydroxypropyl acrylate, 2-hydroxyethyl methacrylate, hydroxypropyl methacrylate, alkylene glycol (meth)acrylates, styrene, unsaturated sulfonic acids such as, for example, acrylamidopropane sulfonic acid, vinylpyrrolidone, vinylcaprolactam, vinyl ethers (e.g.: methyl, ethyl, butyl or dodecyl vinyl ether), vinylformamide, vinylmethylacetamide, vinylamine, 1-vinylimidazole, 1-vinyl-2-methylimidazole, N,N-dimethylaminomethyl methacrylate and N-[3-(dimethylamino)propyl]methacrylamide; 3-methyl-1-vinylimidazolium chloride, 3-methyl-1-vinylimidazolium methylsulfate, N,N-dimethylaminoethyl methacrylate, N-[3-(dimethylamino)propyl]methacrylamide quaternized with methyl chloride, methyl sulfate or diethyl sulfate.

Monomers having one basic nitrogen atom can be quaternized with: alkyl halides having from 1 to 24 carbon atoms in the alkyl group, e.g. methyl chloride, methyl bromide, methyl iodide, ethyl chloride, ethyl bromide, propyl chloride, hexyl chloride, dodecyl chloride, lauryl chloride and benzyl halides, in particular benzyl chloride and benzyl bromide. Other suitable quaternizing agents are dialkyl sulfates, in particular dimethyl sulfate or diethyl sulfate. The quaternization of the basic amines can also be carried out with alkylene oxides such as ethylene oxide or propylene oxide in the presence of acids. Preferred quaternizing agents are: methyl chloride, dimethyl sulfate or diethyl sulfate.

The quaternization can, in addition, be carried out before or after polymerization.

Furthermore, it is possible to use the product derived from reacting unsaturated acids, for example, acrylic or methacrylic acid, with a quaternized epichlorohydrin of general formula (VI) (R²⁶ = C₁ to C₄₀ -alkyl) .

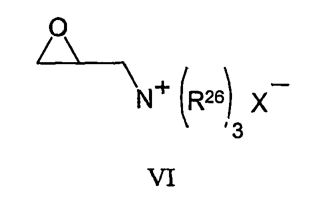

Examples thereof are:
(meth)acryloyloxyhydroxypropyltrimethylammonium chloride and
(meth)acryloyloxyhydroxypropyltriethylammonium chloride.

The basic monomers can also be cationized, by neutralizing them with mineral acids, for example, sulfuric acid, hydrochloric acid, hydrobromic acid, hydroiodic acid, phosphoric acid or nitric acid, or with organic acids, for example, formic acid, acetic acid, lactic acid, or citric acid.

In addition to the abovementioned comonomers, it is also possible to select as comonomer (c), so-called macromonomers, such as, for example, silicone-containing macromonomers having one or more free-radically polymerizable groups or alkyloxazoline macromonomers, as described, for example, in EP 408 311.

Furthermore, it is possible to use fluorine-containing monomers, as described, for example, in EP 558423 and cross-linking compounds or compounds which regulate the molecular weight, in combination or alone.

Regulators which can be used are the customary compounds known to the person skilled in the art, such as, for example, sulfur compounds (e.g. mercaptoethanol, 2-ethylhexyl thioglycolate, thioglycolic acid or dodecylmercaptan), and tribromochloromethane and other compounds which have a regulating effect on the molecular weight of the resulting polymers.

In some instances, it is also possible to use thio-containing silicone compounds. Preference is given, however, to using silicone-free regulators.

Suitable cross-linking monomers, in addition to those mentioned above, are compounds which possess at least two ethylenically unsaturated double bonds, and include, for example, esters derived from ethylenically unsaturated carboxylic acids, such as acrylic or methacrylic acid, and polyhydric alcohols, ethers of at least dihydric alcohols such as, for example, vinyl ethers or allyl ethers. Also suitable are straight-chain or branched, linear or cyclic aliphatic or aromatic hydrocarbons possessing at least two double bonds which, in the case of the aliphatic hydrocarbons, must not be conjugated. Also suitable are amides of acrylic and methacrylic acid and N-allylamines of at least difunctional amines, for example, 1,2-diaminoethane and 1,3-diaminopropane. Also suitable are triallylamine or corresponding ammonium salts, N-vinyl compounds of urea derivatives, at least difunctional amides, cyanurates or urethanes. Other suitable cross-linkers are divinyldioxane, tetraallylsilane or tetravinylsilane.

Particularly preferred cross-linkers are, for example, methylenebisacrylamide, triallylamine and triallylammonium salts, divinylimidazole, N,N'-divinylethyleneurea, reaction products of polyhydric alcohols with acrylic, or methacrylic acid, methacrylic esters and acrylic esters of polyalkylene oxides or polyhydric alcohols which have been reacted with ethylene oxide and/or propylene oxide and/or epichlorohydrin.

It is also possible, in some instances, for other polymers, such as, for example, polyamides, polyurethanes, polyesters, homo- and copolymers of ethylenically unsaturated monomers, to be present during the polymerisation for preparation of the polymers according to the invention. Examples of such polymers, some of which are also used in cosmetics, are the polymers known under the tradenames Amerhol^{™}, Ultrahold^{™}, Ultrahold Strong^{™}, LuvifleX^{™}, VBM, Luvimer^{™}, Acronal^{™}, Acudyne^{™}, Stepanhold^{™}, Lovocryl^{™}, Versatyl^{™}, Amphomer^{™} or Eastma AQ^{™} .

The (c) comonomers according to the invention can, provided they contain ionizable groups, be partially or completely neutralized using acids or bases before or after the polymerization in order, for example, to adjust the solubility or dispersibility in water to the desired degree.

Examples of suitable neutralizing agents for monomers carrying acidic groups are: mineral bases such as sodium carbonate, alkali metal hydroxides and ammonia; organic bases such as aminoalcohols, specifically 2-amino-2-methyl-1-propanol, monoethanolamine, diethanolamine, triethanolamine, triisopropanolamine, tri[(2-hydroxy)-1-propyl]amine, 2-amino-2-methyl-1,3-propanediol, 2-amino-2-hydroxymethyl-1,3-propanediol and diamines, such as, for example, lysine.

To prepare the polymers, the monomers of component a) can be polymerized in the presence of the polyethers using initiators which form free radicals, or by the action of high-energy radiation, which is also intended to mean the action of high-energy electrons.

Initiators which can be used for free-radical polymerization are the customary peroxo and/or azo compounds used for this purpose, and include, for example: alkali metal or ammonium peroxydisulfates, diacetyl peroxide, dibenzoyl peroxide, succinyl peroxide, di-tert-butyl peroxide, tert-butyl perbenzoate, tert-butyl perpivalate, tert-butyl permaleate, cumene hydroperoxide, di-isopropyl peroxydicarbamate, bis-(o-toluoyl) peroxide, didecanoyl peroxide, dioctanoyl peroxide, dilauroyl peroxide, tert-butyl perisobutyrate, tert-butyl peracetate, di-tert-amyl peroxide, tert-butyl hydroperoxide, azobisisobutyronitrile, azobis-(2-amidinopropane) dihydrochloride or 2,2'-azobis(2-methylbutyronitrile). Also suitable are initiator mixtures or redox initiator systems, such as, for example, ascorbic acid/iron(II) sulfate/sodium peroxodisulfate, tert-butyl hydroperoxide/sodium disulfite, tert-butyl hydroperoxide/sodium hydroxymethanesulfinate.

Preference is given to using organic peroxides.

The amounts of initiator or initiator mixtures used, based on monomer used, are between 0.01 and 10% by weight, preferably between 0.1 and 5% by weight.

The polymerization is carried out in a temperature range from 40 to 200°C, preferably in the range from 50 to 140°C, particularly preferably in the range from 60 to 110°C. It is usually carried out under atmospheric pressure, but can also be carried out under reduced or increased pressure, preferably between 1 and 5 bar.

The polymerization can, for example, be carried out as solution polymerization, bulk polymerization, emulsion polymerization, inverse emulsion polymerization, suspension polymerization, inverse suspension polymerization or precipitation polymerization, without the possible methods being limited thereto.

In the case of bulk polymerization, the procedure may involve dissolving the polyether-containing compound b) in at least one monomer of group a) and other comonomers of group c) and, after the addition of a polymerization initiator, fully polymerizing the mixture. The polymerization can also be carried out semi-continuously by firstly introducing some, e.g. 10%, of the mixture to be polymerized, comprising the polyether-containing compound b), at least one monomer from group a), other comonomers of group c) and initiator, heating the mixture to the polymerization temperature and after the polymerization has started, adding the remainder of the mixture to be polymerized in accordance with the progress of the polymerization. The polymers can also be obtained by initially introducing the polyether-containing compounds of group b) into a reactor, heating them to the polymerization temperature and adding at least one monomer of group a), possibly other comonomers of group c) and polymerization initiator either in one portion, step by step or, preferably, continuously, and polymerizing.

If desired, the above described polymerization can also be carried out in a solvent. Suitable solvents are, for example, alcohols, such as methanol, ethanol, n-propanol, iso-propanol, n-butanol, sec-butanol, tert-butanol, n-hexanol and cyclohexanol, and glycols, such as ethylene glycol, propylene glycol and butylene glycol, and the methyl or ethyl ethers of dihydric alchols, diethylene glycol, triethylene glycol, glycerol and dioxane. The polymerization can also be carried out in water as solvent. In this case, the initial batch is a solution which, depending on the amount of monomers of component a) added, is soluble in water to a greater or lesser degree. In order to convert water-insoluble products, which can form during the polymerization, into solution, it is possible, for example, to add organic solvents, such as monohydric alcohols having from 1 to 3 carbon atoms, acetone or dimethylformamide. However, in the case of polymerization in water, it is also possible to convert the water-insoluble polymers into a finely divided dispersion by addition of customary emulsifiers or protective colloids, e.g. polyvinyl alcohol.

The emulsifiers used are, for example, ionic or non-ionic surfactants whose HLB value is in the range from 3 to 13.

The definition of the HLB value can be found in the publication by W.C. Griffin, J. Soc. Cosmetic Chem., Volume 5, 249 (1954).

The amount of surfactants, based on the polymer, is from 0.1 to 10% by weight. Using water as solvent gives solutions or dispersions of the polymers. If solutions of the polymer are prepared in an organic solvent, or in mixtures of an organic solvent and water, then, per 100 parts by weight of the polymer, from 5 to 2000, preferably from 10 to 500, parts by weight of the organic solvent or of the solvent mixture are used.

Preference is given to polymers obtainable by free-radical polymerization of
a) 10 - 98 % by weight of at least one vinyl ester of C₁ C₂₄ carboxylic acids in the presence of
b) 2 - 90 % by weight of at least one polyether-containing compound and
c) 0 - 50 % by weight of one or more other copolymerizable monomers.

Particular preference is given to polymers obtainable by free-radical polymerization of
a) 50 - 97 % by weight of at least one vinyl ester of C₁ - C₂₄ carboxylic acids in the presence of
b) 3 - 50 % by weight of at least one polyether-containing compound and
c) 0 - 30 % by weight of one or more other copolymerizable monomers.

Very particular preference is given to polymers obtainable by free-radical polymerization of
a) 65 - 97 % by weight of at least one vinyl ester of C₁ - C₂₄ carboxylic acids in the presence of
b) 3 - 40 % by weight of at least one polyether-containing compound and
c) 0 - 20 % by weight of one or more other copolymerizable monomers.

To prepare the polymers used according to the invention, the ester groups of the original monomers a) and optionally of other monomers are cleaved after the polymerization by hydrolysis, alcoholysis, or aminolysis. This process step is generally referred to below as hydrolysis. The hydrolysis takes place in a manner known per se by the addition of a base, preferably the addition of a sodium or potassium hydroxide solution in water and/or alcohol. Particular preference is given to using methanolic sodium or potassium hydroxide solutions. The hydrolysis is carried out at temperatures in the range from 10 to 80°C, preferably in the range from 20 to 60°C. The degree of hydrolysis depends on the amount of base used, on the hydrolysis temperature, on the hydrolysis time and the water content of the solution.

The degree of hydrolysis of the polyvinyl ester groups is in the range from 1 to 100%, preferably in the range from 40 to 100%, particularly preferably in the range from 65 to 100%, very particularly preferably in the range from 80 to 100%.

The polymers prepared in this way can then be cationized by reaction of hydroxyl and/or amino functions present in the polymer with epoxides of the formula VI (R²⁶ = C₁ - to C₄₀ - alkyl).

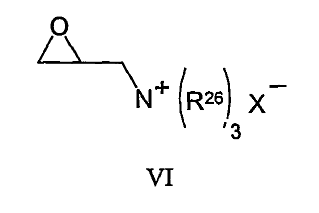

For this, the hydroxyl groups of the polyvinyl alcohol units and vinylamine units, formed by hydrolysis of vinylformamide, can preferably be reacted with the epoxides.

The epoxides of formula VI can also be produced in situ by reaction of the corresponding chlorohydrins with bases, for example sodium hydroxide.

Preference is given to using 2,3-epoxypropyltrimethylammonium chloride or 3-chloro-2-hydroxypropyltrimethylammonium chloride. The K values of the polymers should be in the range from 10 to 300, preferably 25 to 250, particularly preferably 25 to 200, very particularly preferably in the range from 30 to 150. The K value desired in each case can be adjusted in a manner known per se through the composition of the feed substances. The K values are determined in accordance with Fikentscher, Cellulosechemie, Vol. 13, p. 58 to 64, and 71 to 74 (1932) in N-methylpyrrolidone at 25°C and polymer concentrations which, depending on the K value range, are between 0.1 % by weight and 5 % by weight.

After the hydrolysis, the polymer solutions can be steam distilled to remove any solvents. After the steam distillation, aqueous solutions or dispersions are obtained depending on the degree of hydrolysis, type of (b) polyethers, of group (a) vinyl esters and any group (c) monomers used.

The polymer solutions and dispersions can be converted into powder form by a variety of drying methods, such as, for example, spray drying, fluidized spray drying, drum drying or freeze drying. The drying method used in preference is spray drying. The dry polymer powder obtained in this way can be used to prepare an aqueous solution or dispersion again, by dissolution or redispersion in water. Conversion into powder form has the advantage of improved storability, easier transportation, and a lower propensity for microbial attack.

Instead of the steam distilled polymer solutions, the alcoholic polymer solutions can be directly converted into powder form.

### Cationic Hair Styling Polymer

The compositions of the invention comprise, in addition to the copolymer (i), from 0.01% to 10% by weight (based on weight of the total composition) of a cationic hair styling polymer (ii) which may be of the type conventionally used in hair styling compositions. The cationic hair styling polymer which is employed in compositions of the present invention should be capable of forming a film and holding the hair of the user in place.

The cationic hair styling polymer is preferably present in the compositions in an amount of from 0.1% to 5%, more preferably 1% to 2%, such as about 1.5%, by weight based on total weight of the composition.

Cationic hair styling polymers are well known articles of commerce and many such polymers are available commercially.

The compositions of the invention may comprise a single cationic hair styling polymer or a mixture of two or more different cationic hair styling polymers.

Examples of cationic hair styling polymers are copolymers of amino-functionalised acrylate monomers such as lower alkylaminoalkyl acrylate or methacrylate monomers such as dimethylaminoethyl methacrylate with compatible monomers such as N-vinylpyrrolidone, vinyl caprolactam, or alkyl methacrylates such as methyl methacrylate and ethyl methacrylate and alkyl acrylates such as ethyl acrylate and n-butyl acrylate. Cationic hair styling polymers containing N-vinylpyrrolidone are commercially available from ISP Corporation such as those sold under the trademarks of Gafquat^{®} 734, 755 and 755N (quaternary ammonium polymers formed by the reaction of diethyl sulfate and a copolymer of N-vinylpyrrolidone and dimethylaminoethyl methacrylate and having the CTFA designation Polyquaternium-11). Polyquaternium-11 polymers, such as Gafquat 755, are particularly preferred for use in the present invention.

The cationic hair styling polymer preferably has a weight average molecular weight of from 500 Da to 2000 kDa, more preferably from 800 to 1500 kDa, even more preferably from 100kDa to 1500 kDa, such as from 800kDa to 1500 kDa. Molecular weight of the cationic hair styling polymer can be determined by methods well-known to those skilled in the art, including gel permeation chromatography (GPC) as described in the examples section herein.

### Optional Cross-linked Silicone Polymer

A non-rigid emulsion-polymerised cross-linked silicone polymer (iii) is optionally present in compositions of the invention in an amount of less than 0.1% by weight based on the total weight of the composition, preferably less than 0.01% by weight. Most preferably, the compositions are free or substantially free of non-rigid emulsion-polymerised cross-linked silicone polymer.

Preferred silicone polymers for use in the invention are polydiorganosiloxanes, preferably derived from suitable combinations of R₃SiO_{0.5} units and R₂SiO units where each R independently represents an alkyl, alkenyl (e.g., vinyl), alkaryl, aralkyl, or aryl (e.g. phenyl) group. R is most preferably methyl.

The preferred silicone polymers of the invention are cross-linked polydimethyl siloxanes (which have the CTFA designation dimethicone), and cross-linked polydimethyl siloxanes having end groups such as hydroxyl (which have the CTFA designation dimethiconol). Good results have been obtained with cross-linked dimethiconol.

Cross-linking of the silicone polymer is typically introduced concurrently during emulsion polymerisation of the polymer through the inclusion of the required amount of trifunctional and tetrafunctional silane monomer units, for example, those of formula:
R Si (OH)₃ wherein R represents an alkyl, alkenyl (e.g. vinyl), alkaryl, aralkyl or aryl (e.g. phenyl) group, preferably methyl.

The degree of cross-linking of the silicone polymer can be measured as the percentage of branched monomer units in the silicone polymer and is from 0.05% to 10%, preferably being in the range 0.15% to 7%, e.g. from 0.2% to 2%. It will be understood by those skilled in the art that the percentage of branched monomer units in the silicone polymer is a percentage by weight. Increasing cross-linking is found to improve styling benefits but also to reduce conditioning performance somewhat, so compromise levels must be selected with properties optimised to suit consumer preferences in different cases. Good overall performance has been obtained with dimethiconol 0.3% cross-linked.

Suitable emulsion polymerised cross-linked silicone polymers are commercially available or can be readily made using conventional techniques well known to those skilled in the art.

Cross-linked silicone polymers are described in EP 818190.

### Water

Compositions of the present invention will also include water, preferably distilled or de-ionised, as a solvent or carrier for the polymers and other components. Water will typically be present in amounts ranging from 30% to 98%, preferably from 60% to 95% by weight based on total weight.

Alcohol may optionally be employed as a co-solvent in compositions of the invention as this can enhance the performance of the styling composition. A suitable alcohol is an aliphatic straight or branched chain monohydric alcohol having 2 to about 4 carbon atoms. Isopropanol and especially ethanol are preferred. A suitable level for the alcohol is up to 20%, preferably from 5% to 15%, by weight based on total weight. Preferred compositions of the invention, however, are free or substantially free of ethanol.

### Optional Further Hair Styling Polymers

Compositions of the present invention may, optionally, comprise anionic, amphoteric, or non-ionic hair styling polymers of the type conventionally used in the hair styling compositions.

Examples of anionic hair styling polymers are the copolymers of vinyl acetate and crotonic acid, terpolymers of vinyl acetate, crotonic acid and a vinyl ester of an alpha-branched saturated aliphatic monocarboxylic acid such as vinyl neodecanoate; copolymers of methyl vinyl ether and maleic anhydride (molar ratio about 1:1) wherein such copolymers are 50% esterified with a saturated alcohol containing from 1 to 4 carbon atoms such as ethanol or butanol; and acrylic copolymers, terpolymers, etc., containing acrylic acid or methacrylic acid as the anionic radical-containing moiety and esters of acrylic or methacrylic acid with one or more saturated alcohols having from 1 to 22 carbon atoms such as methyl methacrylate, methyl acrylate, ethyl acrylate, ethyl methacrylate, n-butyl acrylate, t-butyl acrylate, t-butyl methacrylate, n-butyl methacrylate, n-hexyl acrylate, n-octyl acrylate, lauryl methacrylate and behenyl acrylate, glycols having from 1 to 6 carbon atoms such as hydroxypropyl methacrylate and hydroxyethyl acrylate, styrene, vinyl caprolactam, vinyl acetate, acrylamide, alkyl acrylamides and methacrylamides having 1 to 8 carbon atoms in the alkyl group such as methacrylamide, t-butyl acrylamide and n-octyl acrylamide, and other compatible unsaturated monomers. One specific example is the emulsion polymerised terpolymer of methacrylic acid, n-butyl acrylate and ethyl acrylate (e.g., in a weight percent ratio of 31:42:27, respectively). Another specific example is the Gantrez^{®} ES series commercially available from ISP corporation (esterified copolymers of methyl vinyl ether and maleic anhydride).

Examples of amphoteric hair styling polymers are those which contain cationic groups derived from monomers such as t-butyl aminoethyl methacrylate as well as carboxyl groups derived from monomers such as acrylic acid or methacrylic acid. One specific example of an amphoteric hair styling polymer is Amphomer^{®} sold by the National Starch and Chemical Corporation.

Examples of non-ionic hair styling polymers are homopolymers of N-vinylpyrrolidone and copolymers of N-vinylpyrrolidone with compatible non-ionic monomers such as vinyl acetate and terpolymers of ethyl acrylate, butyl methacrylate and methyl methacrylate. Non-ionic polymers containing N-vinylpyrrolidone in various weight average molecular weights are available commercially from ISP Corporation such as those sold under the trade marks Copolymer 845 and Copolymer 937 (copolymers of N-vinylpyrrolidone and t-butylaminoethyl methacrylate of average molecular weight about 1,000,000) and homopolymers of N-vinylpyrrolidone having an average molecular weight of about 630,000 sold by ISP Corporation under the tradename PVP K-90 and those having an average molecular weight of about 1,000,000 sold under the tradename PVP K-120.

The further hair styling polymers in compositions of the invention are most preferably selected from amphoteric and/or non-ionic hair styling polymers. Such non-ionic hair styling polymers are preferably selected from vinylpyrrolidone homopolymers and especially copolymers of vinylpyrrolidone and vinyl acetate.

### Optional Nonionic Surfactant

In addition to the cross-linked silicone polymer and the hair styling polymer, the hair styling composition of the invention may, optionally, also include a non-ionic surfactant in an amount of up to 5%, preferably from 0.01% to 1%, most preferably from 0.02% to 0.8% by weight based on total weight.

The HLB (hydrophilic-lipophilic balance) is an important property of the non-ionic surfactant. The property *per se* and how it is calculated is described in J.Soc.Cosmet.Chem.,1949,1,311*.* For a given non-ionic surfactant, the HLB value represents the weight per cent of the hydrophilic content of the molecule divided by a factor of five.

Non-ionic surfactants for use in compositions of the invention typically have an HLB (hydrophilic - lipophilic balance) value of at least 14.5. The HLB value preferably ranges from 15 to 19, most preferably from 16 to 18.

Examples of suitable non-ionic surfactants are condensation products of aliphatic (C₈-C₁₈) primary or secondary linear or branched chain alcohols or phenols with alkylene oxides, usually ethylene oxide and generally having at least 15, preferably at least 20, most preferably from 30 to 50 ethylene oxide groups. Other suitable non-ionics include esters of sorbitol, esters of sorbitan anhydrides, esters of propylene glycol, fatty acid esters of polyethylene glycol, fatty acid esters of polypropylene glycol, ethoxylated esters and polyoxyethylene fatty ether phosphates.

Of particular use are those non-ionic surfactants of general formula R(EO)ₓ H, where R represents a straight or branched chain alkyl group having an average carbon chain length of 12-18 carbon atoms and x ranges from 30 to 50. Specific examples include steareth-40, steareth-50, ceteareth-30, ceteareth-40, ceteareth-50 and mixtures thereof. Suitable commercially available examples of these materials include Unicol SA-40 (Universal Preserv-A-Chem), Empilan KM50 (Albright and Wilson), NONION PS-250 (Nippon Oils & Fats), Volpo CS50 (Croda Inc), and Incropol CS-50 (Croda Inc).

### Hair Conditioning Agents

Hair conditioning agents such as hydrocarbons, silicone fluids, and cationic materials may optionally be included in the compositions of the invention. Hair conditioning agents may typically be present in compositions of the invention in amounts of from 0.001% to 10% by weight, preferably 0.1% to 1% by weight. Hair conditioning agents may be single compounds or mixtures of two or more compounds from the same class or different general classes.

Suitable hydrocarbons can be either straight or branched chain and can contain from about 10 to about 16, preferably from about 12 to about 16 carbon atoms. Examples of suitable hydrocarbons are decane, dodecane, tetradecane, tridecane, and mixtures thereof.

Examples of suitable silicone conditioning agents useful herein can include either cyclic or linear polydimethylsiloxanes, phenyl and alkyl phenyl silicones, and silicone copolyols. Cationic conditioning agents useful herein can include quaternary ammonium salts or the salts of fatty amines, such as cetyl ammonium chloride, for example.

Compositions according to the invention may, optionally, comprise from 0.1% to 10% by weight of a volatile silicone as the hair conditioning agent. Volatile silicones are well known in the art and are commercially available and include, for example linear and cyclic compounds. Volatile silicone oils are preferably linear or cyclic polydimethylsiloxanes containing from about three to about nine silicon atoms.

Preferred silicone polymers for use in the invention are polydiorganosiloxanes, preferably derived from suitable combinations of R₃SiO_{0.5} units and R₂SiO units where each R independently represents an alkyl, alkenyl (e.g., vinyl), alkaryl, aralkyl, or aryl (e.g. phenyl) group. R is most preferably methyl.

### Product Form

Compositions of the invention may suitably be in aerosol form. A particularly preferred product form is an aerosol hair mousse. Aerosol hair mousse compositions are emitted from the aerosol container as a foam which is then typically worked through the hair with fingers or a hair styling tool and either left on the hair or rinsed out.

Aerosol-form compositions of the invention will include an aerosol propellant which serves to expel the other materials from the container, and forms the mousse character in mousse compositions. The aerosol propellant included in styling compositions of the present invention can be any liquefiable gas conventionally used for aerosol containers. Examples of suitable propellants include dimethyl ether and hydrocarbon propellants such as propane, n-butane and iso-butane. The propellants may be used singly or admixed. Water insoluble propellants, especially hydrocarbons, are preferred because they form emulsion droplets on agitation and create suitable mousse foam densities. A particularly preferred propellant comprises dimethyl ether and at least one hydrocarbon and, more preferably, the weight ratio of dimethyl ether to total hydrocarbon is in the range of from 80:20 to 40:60.

The amount of the propellant used is governed by normal factors well known in the aerosol art. For mousses the level of propellant is generally up to 30%, preferably from 2% to 30%, most preferably from 3% to 15% by weight based on total weight of the composition. If a propellant such as dimethyl ether includes a vapour pressure suppressant (e.g. trichloroethane or dichloromethane), for weight percentage calculations, the amount of suppressant is included as part of the propellant.

The method of preparing aerosol hair styling mousse compositions according to the invention follows conventional aerosol filling procedures. The composition ingredients (not including the propellant) are charged into a suitable pressurisable container which is sealed and then charged with the propellant according to conventional techniques.

Compositions of the invention may also take a non-foaming product form, such as a hair styling cream or gel. Such a cream or gel will include a structurant or thickener, typically at a level of from 0.1% to 10%, preferably 0.5% to 3% by weight based on total weight.

Examples of suitable structurants or thickeners are polymeric thickeners such as carboxyvinyl polymers. A carboxyvinyl polymer is an interpolymer of a monomeric mixture comprising a monomeric olefinically unsaturated carboxylic acid, and from about 0.01% to about 10% by weight of the total monomers of a polyether of a polyhydric alcohol. Carboxyvinyl polymers are substantially insoluble in liquid, volatile organic hydrocarbons and are dimensionally stable on exposure to air. Suitably the molecular weight of the carboxyvinyl polymer is at least 750,000, preferably at least 1,250,000, most preferably at least 3,000,000. Preferred carboxyvinyl polymers are copolymers of acrylic acid cross-linked with polyallylsucrose as described in US Patent 2,798,053. These polymers are provided by B.F.Goodrich Company as, for example, CARBOPOL 934, 940, 941 and 980. Other materials that can also be used as structurants or thickeners include those that can impart a gel-like viscosity to the composition, such as water soluble or colloidally water soluble polymers like cellulose ethers (e.g. methylcellulose, hydroxyethylcellulose, hydroxypropylmethylcellulose and carboxymethylcellulose), guar gum, sodium alginate, gum arabic, xanthan gum, polyvinyl alcohol, polyvinyl pyrrolidone,hydroxypropyl guar gum, starch and starch derivatives, and other thickeners, viscosity modifiers, gelling agents, etc. It is also possible to use inorganic thickeners such as bentonite or laponite clays.

The hair styling compositions of the invention can contain a variety of non-essential, optional components suitable for rendering the compositions more aesthetically acceptable or to aid use, including discharge from the container, of the product. Such conventional optional ingredients are well known to those skilled in the art, e.g. preservatives such as benzyl alcohol, methyl paraben, propyl paraben and imidazolidinyl urea, fatty alcohols such as cetearyl alcohol, cetyl alcohol and stearyl alcohol, pH adjusting agents such as citric acid, succinic acid, sodium hydroxide and triethanolamine, colouring agents such as any of the FD&C or D&C dyes, perfume oils, chelating agents such as ethylenediamine tetraacetic acid, and polymer plasticising agents such as glycerin and propylene glycol.

The invention also provides a method of styling hair by applying thereto a styling composition as is hereinabove described.

The following Examples further illustrate the preferred embodiments of the invention. All percentages referred to throughout this specification are by weight based on total weight unless otherwise indicated. Concentrations are based on 100% active ingredient.

### Examples

### Method for Characterising the Polydispersity of an aqueous sample of PEG/VOH

### SCOPE

The molecular weight distributions and polydispersities of PEG/VOH (polyethylene glycol/polyvinyl alcohol copolymers) are determined relative to polyethylene glycol/polyethylene oxide (PEG/PEO) using aqueous size exclusion chromatography with refractive index detection (SEC-RI).

### PRINCIPLE

The PEG/PEO molecular weight standards and samples are prepared in mobile phase and analysed by aqueous SEC-RI. The polymer species are separated based on their molecular size and their molecular weight distributions (Mₙ and Mw) and polydispersity (M_{w}/Mₙ) are then calculated relative to a 5^{th} order calibration plot of the mono-dispersive PEG/PEO molecular weight standards.

### APPARATUS

High performance liquid chromatography system -
510 Pump (Waters Corporation)
WISP 717 Plus Autosampler (Waters Corporation)
Optilab DSP Inteferometric Refractometer (Wyatt Technology Corporation)
Column Heater & Control Module (Waters Corporation) Millennium Chromatography Client Server with GPC Option
(Waters Corporation)
In-line solvent degassing module - (Alltech)

### SEC columns -

TSK Gel G5000PWXL, (1), 10µm particle size, 1000Å pore size, 7.8 mm x 300 mm (Tosoh Biosep)
TSK Gel G3000PWXL, (1), 6µm particle size, 200Å pore size, 7.8 mm x 300 mm (Tosoh Biosep)
TSK Gel G2500PWXL, (2), 6µm particle size, <200Å pore size, 7.8 mm x 300 mm (Tosoh Biosep)

### pH meter

Magnetic stirrer
Ultrasonic bath
Scintillation vials - glass, 20 mL, polypropylene lined caps Syringe filters - 13 mm, 0.45 µm pore size, PTFE (Whatman) Graduated cylinder - 2 L
Vacuum filter/degassing apparatus (Alltech)
Filter membranes - 0.2 µm pore size, 47 mm diameter, Nylon 66 (Alltech)

### REAGENTS

Water - HPLC grade (Milli-Q system by Millipore Corporation) Sodium chloride - Certified ACS grade (Fisher Scientific) Tris (hydroxymethyl) aminomethane - Certified ACS grade (Fisher Scientific)
Hydrochloric acid - concentrated, 36.5-38%, J.T. Baker Analyzed Reagent grade
PEO-10 calibration kit containing 0.2g of 10 monodispersive polyethylene oxide standards ranging from 1 million to 20000 g/mole (Polymer Laboratories)
PEG-10 calibration kit containing 0.5g of 10 monodispersive polyethylene glycol standards ranging from 22,000 to 106 g/mole (Polymer Laboratories)

### PROCEDURE

A. Preparation of Mobile Phase: (0.08M Tris/0.15M Sodium Chloride in Water, pH 7)
   1. Transfer 19.4 g of tris (hydroxymethyl) aminomethane and 17.6 g of sodium chloride into a 2 L solvent reservoir.
   2.Add 2 L of HPLC grade water using a graduated cylinder and mix well.
   3. Adjust the pH of the mobile phase to pH 7 by first adding about 5 mL of concentrated hydrochloric acid to reservoir while mixing. Continue to adjust the pH to 7 using 50% hydrochloric acid, aqueous.
   4. Filter/degas the mobile phase under vacuum through the 0.2 µm Nylon 66 membrane into another solvent reservoir.
B. Preparation of PEG/PEO Molecular Weight Standards
   1. Prepare 5 vials of mixed PEG/PEO standards (ranging from ca. 280000 to 440 g/mole) at concentrations of 0.1 wt% each in mobile phase. Vial 1 (288000, 32600, and 1470), Vial 2 (205000, 18200, and 1080), Vial 3 (120000, 10000, and 620), Vial 4 (73400, 7100, and 440), Vial 5 (50100 and 4120). Notes: Although the TSK G5000PWXL column extends the linear working range to ca. 1 million g/mole, the PEO standards >288000 tend to exhibit nonlinear behaviour due to peak broadening. This effect is suspected to be caused by the high salt/buffer content of the mobile phase which is required for analyzing the PEG/VOH. Typically, the PEG/PEO standards are linear through about 1 million g/mole when analyzed in 100% water or in water containing up to about 0.1M sodium nitrate.
   2. Filter the solutions through 0.45 µm PTFE syringe filters into autosampler vials for analysis.
C. Preparation of Sample
   1. The sample weight to be used is determined based on the polymer concentration of the sample. The sample solution should have a polymer concentration of about 0.2 wt%. Weigh the sample into a 20 mL scintillation vial, add 10 mL of filtered mobile phase, and mix well. The sample solution should be allowed to stand at room temperature for about 1 hour.
   2. Place the vial into the ultrasonic bath for 30 seconds, then transfer the sample solution into an autosampler vial for analysis. The ultrasonic bath is used to breakup polymer aggregates which may have formed in the solution. Preliminary analyses also indicated the potential for some polymer loss when passed through a 0.45 µm PTFE syringe filter, therefore the sample solution should be analyzed unfiltered. Note: Injecting unfiltered samples may cause some loss in column resolution over time. It is recommended that a sample be selected for use as a control and analyzed before and after a large sample set to monitor column performance. If a loss in resolution occurs, the mobile phase should be slowly switched over to 50 wt% acetonitrile in water and then allowed to run at a flow rate of 1 mL/min. Depending on the degree of contamination, the column set should be flushed with about 250 to 500 mL of mobile phase.

### CHROMATOGRAPHY

Set up the SEC system using the following parameters: Columns:TSK Gel G5000PWXL, G3000PWXL, G2500PWXL, and G2500PWXL in series

| | |
|---|---|
| Column oven temperature: | 37°C |
| Flow rate: | 1 mL/min |
| Optilab DSP temperature: | 37°C |
| Injection volume: | 200 µL |
| Total run time: | 60 min |

### CALCULATIONS

### 1. Narrow PEG/PEO Calibration Method

Note: Refer to the manufacturer's instruction manual on how to perform narrow SEC calibrations using a fifth order fit.
The Mp value of each PEG/PEO standard calculated from the fifth order calibration curve should possess a standard deviation of less than 2%. It may be necessary to remove some of the calibration points which have high standard deviations to achieve this degree of precision, however the 288000 PEO standard should not be removed. Typically, the 120000, 73400, 18200, and 1000 g/mole calibration points often possess higher standard deviations.

### Examples 1 to 5

The following copolymers that are useful in the present invention are obtainable according to the procedure outlined in WO 00/49998 (in particular, Examples 29 and 30 in that document).

All copolymers were based on 35K backbone PEG, 50:50 weight ratio with poly(vinyl alcohol) grafts, 0.4% by weight cross-linker (pentaerythritol triallyl ether). The copolymers were determined as having the following molecular weight properties:

| | Example 1 | Example 2 | Example 3 | Example 4 | Example 5 |
|---|---|---|---|---|---|
| M_{W} (Da) | 316470 | 570000 | 188000 | 304000 | 421000 |
| Mₙ (Da) | 16000 | 35000 | 13500 | 14300 | 15000 |
| Polydispersity | 19,7 | 16,3 | 13,9 | 21,3 | 28,1 |

### Example 6

### In-Vitro Evaluation of PEG/VOH Polymers

### Test

The results reported below are a comparison of the styling performance of mousses assessed by an expert stylist on mannequin head wiglets. The mannequin head is shaved such that only the hair on the upper half of the head is used. This allows better differentiation of styling attributes such as volume and root lift. The head was washed in a standard shampoo. One side of the hair was styled with a benchmark mousse (the commercially available Thermasilk Volumizing Mousse from Helene Curtis) and the other with a prototype mousse. The expert assessor rated the prototype against the benchmark for the styling attributes listed in table 2 on a 10 point scale. If there was no difference between the prototype and the standard the score was 5 (parity). The hair was styled using a brush and a hair dryer. The stylist tested the products blind using only codes to identify each formula.

### Formulations for test:

Two formulations are described in table 1. The levels are weight% of active material. The propellant CAP 40 is from Calor Gas. Volpo CS50 is a surfactant from Croda Chemicals. Gafquat 755N is sourced from Gaf Chemicals. The PEG/VOH polymer used in formulations A and B differ in the molecular weight of the PEG polymer backbone. The PEG/VOH polymers have a 35K Mw PEG backbone (Formulation A) and a 12K backbone (formulation B). Both polymers were crosslinked using 0.4wt% pentaerythritol triallyl ether. The polydispersity (PD) of each polymer is indicated in brackets in table 1.

**Table 1. Mousse formulations**

| Ingredient | Level in A (wt%) | Level in B (wt%) |
|---|---|---|
| PEG/VOH Polymer | 1.5 (PD=36.1) | 1.5 (PD=2.24) |
| Gafquat 755N | 1.5 | 1.5 |
| Volpo CS50 | 0.3 | 0.3 |
| CAP 40 | 8 | 8 |
| Water | 88.7 | 88.7 |

### Results:

Table 2 is a comparison of the performance of each mousse versus the benchmark formulation. The only difference between the formulations is the PEG/VOH polymer type.

**Table 2. Relative scores of the prototypes versus the benchmark formulation.**

| **Attribute** | **Formulation A** | **Formulation B** | **A-B** |
|---|---|---|---|
| Polydispersity of Polymer | 36.1 | 2.24 | |
| Grip | 7 | 6 | 1 |
| Shaping Power | 7 | 6 | 1 |
| Root Lift | 6 | 4 | 2 |
| Volume | 9 | 6 | 3 |
| Natural Feel | 6 | 4 | 2 |

All of the attributes are scored higher for formula A than formula B.

Attributes explanation:
Grip is the amount that the brush holds the hair on the brush whilst it is being styled. Shaping power is the amount of incongruous shape that it is possible to generate with brush styling. Root lift is the amount of volume it is possible to generate with the hair near the scalp. Volume is the overall total hair-style volume. Natural feel describes how close to untreated hair the styled wiglet feels.

### Examples 7 to 11

The following are examples of mousse formulations of the invention. All figures are percentages are by weight based on total formulation and are based on active material.

| **Ingredient** | **Examples** | | | | |
|---|---|---|---|---|---|
| | 7 | 8 | 9 | 10 | 11 |
| Polymer A | 1.5 | 2 | | | |
| Polymer B | | | 2 | | |
| Polymer C | | | | 1.5 | 1.5 |
| Gafquat 755 | 1.5 | | 1 | | |
| Gafquat 734 | | 2 | | | |
| Luviquat Hold | | | | 1.5 | |
| FC 550 | | | | | 1.5 |
| Volpo CS50 | 0.3 | 0.3 | 0.3 | 0.3 | 0.3 |
| CAP40 | 8 | 8 | 8 | 8 | 8 |
| Ethanol | | 8 | | | |
| Formalin | | 0.1 | | | |
| Water | To 100 | To 100 | To 100 | To 100 | To 100 |

Polymers A, B and C are PEG/VOH polymers having a 35K Mw PEG backbone crosslinked using 0.4wt% pentaerythritol triallyl ether. The polydispersities of Polymers A, B and C are 33.2, 36.1 and 35, respectively.

Luviquat Hold and FC 550 are Polyquaternium-46 and Polyquaternium-16 polymers, respectively, available from BASF.

## Claims

1. Hair styling composition comprising
(i) from 0.01% to 10% by weight of a copolymer having a backbone comprising a polyethyleneglycol and, depending from said backbone, a plurality of poly (vinyl ester) groups, wherein at least 50% of the ester groups are hydrolysed to the corresponding alcohol, and wherein the copolymer is crosslinked and has a polydispersity of at least 5;
(ii) from 0.01% to 10% by weight of a cationic hair styling polymer;
(iii) optionally, a cross linked silicone polymer in an amount of less chin 0.1% by weight; and
(iv) water. ,

2. Composition as claimed in Claim 1, wherein the total amount of (i) and (ii) is less than 10% by weight of the total weight of the composition.

3. Composition as claimed in Claim 1 or Claim 2, wherein the weight ratio of (1) to (ii) is from 10:1 to 1:10.

4. Composition as claimed in Claim 3, wherein the weight ratio of (i) to (ii) is from 2:1 to 1:2.

5. Composition as claimed in any one of Claims 1 to 4, wherein the copolymer has a polydispersity of from 5 to 40.

6. Composition as claimed in any one of Claims 1 to 5, wherein the cationic hair styling polymer is Polyquaternium-11.

7. Composition as claimed in any one of Claims 1 to 6, wherein the copolymer contains from 0.1% to 1% by weight cross-linker.

8. Composition as claimed in any one of Claims 1 to 7, wherein the copolymer is cross-linked using pentaerythritol triallyl ether.

9. Composition as claimed in any one of Claims 1 to 8, wherein the weight ratio of polyether to hydrolysed and unhydrolysed poly(vinyl ester) groups in copolymer (i) is in the range of from 7:3 to 3:7.

10. Composition as claimed in any one of Claims 1 to 9 wherein in copolymer (i) at least 90% by number of the poly(vinyl ester) groups are hydrolysed to poly(vinyl alcohol) groups.

11. Composition as claimed in any one of Claims 1 to 10 which is an aerosol hair mousse formulation further comprising a propellant.

12. Composition as claimed in Claim 11 wherein the propellant comprises dimethyl ether and at least one hydrocarbon.

13. Compositions as claimed in Claim 12 wherein the weight ratio of dimethyl ether to total hydrocarbon is in the range of from 80:20 to 40:60.

14. Method of styling hair which comprises applying to the hair a composition according co any one of Claims 1 to 13.

15. use of a composition according to any one of Claims 1 co 13 for styling hair.

## Patentansprüche

1. Haar-Styling-Zusammensetzung, umfassend
(i) 0,01 Gew.-% bis 10 Gew.-% eines Copolymers, das eine Hauptkette, die ein Polyethylenglykol umfasst, und hervorstehend aus der Hauptkette eine Vielzahl von Poly(vinylester)-Gruppen hat, wobei wenigstens 50 % der Ester-Gruppen zu dem entsprechenden Alkohol hydrolysiert sind und wobei das Copolymer vernetzt ist und eine Polydispersität von wenigstens 5 hat;
(ii) 0,01 Gew.-% bis 10 Gew.-% eines kationischen Hair-Styling-Polymers;
(iii) gegebenenfalls ein vernetztes Siliconpolymer in einer Menge von weniger als 0,1 Gew.-% und
(iv) Wasser.

2. Zusammensetzung nach Anspruch 1, wobei die Gesamtmenge an (i) und (ii) weniger als 10 Gew.-% des Gesamtgewichts der Zusammensetzung ist.

3. Zusammensetzung nach Anspruch 1 oder 2, wobei das Gewichtsverhältnis von (i) zu(ii) 10:1 bis 1:10 ist.

4. Zusammensetzung nach Anspruch 3, wobei das Gewichtsverhältnis von (i) zu (ii) von 2:1 bis 1:2 ist.

5. Zusammensetzung nach einem der Ansprüche 1 bis 4, wobei das Copolymer eine Polydispersität von 5 bis 40 hat.

6. Zusammensetzung nach einem der Ansprüche 1 bis 5, wobei das kationische Haar-Styling-Polymer Polyquaternium-11 ist.

7. Zusammensetzung nach einem der Ansprüche 1 bis 6, wobei das Copolymer 0,1 Gew.-% bis 1 Gew.-% Vernetzungsmittel enthält.

8. Zusammensetzung nach einem der Ansprüche 1 bis 7, wobei das Copolymer unter Verwendung von Pentaerythrritoltriallylether vernetzt ist.

9. Zusammensetzung nach einem der Ansprüche 1 bis 8, wobei das Gewichtsverhältnis von Polyether zu hydrolysierten und unhydrolysierten Poly(vinylester)-Gruppen im Copolymer (i) im Bereich von 7:3 bis 3:7 liegt.

10. Zusammensetzung nach einem der Ansprüche 1 bis 9, wobei in Copolymer (i) wenigstens 90 Zahlenprozent der Poly(vinylester)-Gruppen zu Poly(vinylalkohol)-Gruppen hydrolysiert sind.

11. Zusammensetzung nach einem der Ansprüche 1 bis 10, welche eine Aerosol-Haarschaum-Formulierung ist, die außerdem ein Treibmittel umfasst.

12. Zusammensetzung nach Anspruch 11, wobei das Treibmittel Dimethylether und wenigstens einen Kohlenwasserstoff umfasst.

13. Zusammensetzung nach Anspruch 12, wobei das Gewichtsverhältnis von Dimethylether zu Gesamt-Kohlenwasserstoff im Bereich von 80:20 bis 40:60 ist.

14. Verfahren zum Styling von Haar, umfassend Auftragen einer Zusammensetzung nach einem der Ansprüche 1 bis 13 auf das Haar.

15. Verwendung einer Zusammensetzung nach einem der Ansprüche 1 bis 13 zum Styling von Haar.

## Revendications

1. Composition de coiffure comprenant
(i) de 0,01 % à 10 % en poids d'un copolymère ayant un squelette comprenant un polyéthylène glycol et, en fonction dudit squelette, une pluralité de groupes poly-(vinylester), dans laquelle au moins 50 % des groupes esters sont hydrolysés en l'alcool correspondant, et dans laquelle le copolymère est réticulé et présente une polydispersité d'au moins 5 ;
(ii) de 0,01 % à 10 % en poids d'un polymère de mise en forme des cheveux de type cationique ;
(iii) de manière facultative, un polymère silicone réticulé en une quantité inférieure à 0,1 % en poids; et
(iv) de l'eau.

2. Composition selon la revendication 1, dans laquelle la quantité totale de (i) et (ii) est inférieure à 10 % en poids du poids total de la composition.

3. Composition selon la revendication 1 ou la revendication 2, dans laquelle le rapport pondéral (i)/(ii) varie de 10/1 à 1/10.

4. Composition selon la revendication 3, dans laquelle le rapport pondéral (i)/(ii) varie de 2/1 à 1/2.

5. Composition selon l'une quelconque des revendications 1 à 4, dans laquelle le copolymère possède une polydispersité allant de 5 à 40.

6. Composition selon l'une quelconque des revendications 1 à 5, dans laquelle le polymère de mise en forme des cheveux de type cationique est le Polyquaternium-11.

7. Composition selon l'une quelconque des revendications 1 à 6, dans laquelle le copolymère contient de 0,1 % à 1 % en poids d'un agent réticulant.

8. Composition selon l'une quelconque des revendications 1 à 7, dans laquelle le copolymère est réticulé en utilisant le pentaérythritol tri-allyléther.

9. Composition selon l'une quelconque des revendications 1 à 8, dans laquelle le rapport pondéral entre le polyéther et les groupes poly-(vinylester) hydrolysés et non hydrolysés dans le copolymère (i) se situe dans la plage allant de 7/3 à 3/7.

10. Composition selon l'une quelconque des revendications 1 à 9, dans laquelle, dans le copolymère (i), au moins 90 % en nombre des groupes poly(vinylester) sont hydrolysés en groupes poly(vinylalcool).

11. Composition selon l'une quelconque des revendications 1 à 10, laquelle est une formulation de mousse capillaire en aérosol comprenant en outre un gaz propulseur.

12. Composition selon la revendication 11, dans laquelle le gaz propulseur comprend l'éther diméthylique et au moins un hydrocarbure.

13. Composition selon la revendication 12, dans laquelle le rapport pondéral entre l'éther diméthylique et l'hydrocarbure total se situe dans la plage allant de 80/20 à 40/60.

14. Procédé de mise en forme des cheveux qui comprend l'application sur les cheveux d'une composition selon l'une quelconque des revendications 1 à 13.

15. Utilisation d'une composition selon l'une quelconque des revendications 1 à 13 pour la mise en forme des cheveux.
